# EUROPEAN PATENT APPLICATION

(11) **EP 1 424 372 A1**
(43) Date of publication of application: **02.06.2004**
(21) Application number: 02762753.8
(22) Date of filing: 07.08.2002
(51) Int. Cl.: C09C 3/06

(54) **TITANIUM OXIDE COMPOSITION HAVING HIGH BRILLIANT COLOR, COMPOSITION COMPRISING COATING AND COSMETIC COMPOSITION, AND METHOD FOR THEIR PREPARATION**

(30) Priority: 10.08.2001 JP 2001277257; 07.08.2002 JP 2002263365
(71) Applicant: Nihonkoken Kougyo Kabushiki Kaisha, Tachikawa-shi, Tokyo 190-0033 (JP)
(72) Inventor: KOBAYASHI, Kohta, Edogawa-ku, Tokyo 133-0073 (JP); YANAGITA, Azuma, Akishima-shi, Tokyo 196-0001 (JP); KAIDA, Takahiro, Higashiyamato-shi, Tokyo 207-0013 (JP); NAGIRA, Fumi, Tachikawa-shi, Tokyo 190-0033 (JP); MURUI, Yukio, Sagamihara-shi, Kanagawa 229-0011 (JP); SUZUKI, Fukuji, Atsugi-shi, Kanagawa 243-0207 (JP)
(74) Representative: Hyden, Martin Douglas
(86) International application number: PCT/JP2002/008060
(87) International publication number: WO 2003/016409

(57) **Abstract**

A highly iridescent titanium oxide composition, which not only produces new and outstanding luster as well as color brightness within a recognized color range by interference colors but also retains clear complementary colors. The highly iridescent titanium oxide composition is a coating composition forming a coating layer over the surface of a thin flake-shaped matrix, whose size is 50 - 800µm. The coating layer is 0.05 - 0.6µm thick containing a titanium composition whose content is 70 - 95% by mass. This titanium oxide-contained coating layer is the highly iridescent titanium oxide composition exfoliated from the coating composition. The highly iridescent titanium oxide composition is a beneficial element, especially as a cosmetic ingredient.

## Description

### Field of the invention

The present invention pertains to a highly iridescent titanium oxide composition, or more specifically, it relates to a titanium composition newly produced by the exfoliation of its original titanium coating composition, which is formed over the surface of a thin flake-shaped matrix. The present invention also pertains to a cosmetic composition which particularly contains the aforesaid exfoliated composition. This cosmetic composition, in more detail, not only provides a smooth texture to the skin and natural transparency but also controls pigmentation irregularities (blemish control effect) and is excellent in providing anti-UV and photochromic effects.

### Prior art

Pearl luster pigments are commonly known in the prior art, which are produced by coating natural mica with a metal oxide such as iron oxide, titanium oxide etc. to create the pearl-like effect. However, such conventionally produced pearl luster pigments have disadvantages; they lack brightness in both luster and color, and their complementary colors can be dull and cloudy. Synthetic mica produces highly transparent products, and in this sense, it is right to say that synthetic mica makes an excellent matrix for pearl luster pigments. Consisting of hard crystals, however, synthetic mica is difficult to cleave; thus, it cannot provide a surface as smooth as natural mica can under conventional methods of producing pearl luster pigments. Nevertheless, synthetic mica has a high degree of transparency and whiteness; hence, it became a commonly used material for making matrixes to produce pearl luster pigments which are coated in thin film of titanium oxide.

Synthetic mica has also been used as a pearl luster pigment, which is coated in a thin film of titanium oxide etc., using plate-shaped alumina or silicon oxide for its matrix in order to enhance the photoluminescent effect of interference color.

Meanwhile, making full use of its characteristics; whiteness and UV-shielding performance, titanium oxide has been a widely used additive for paints and cosmetics as well as resins and paper. However, conventionally manufactured equal-sized particles in these products have always raised questions about the spreadability, adhesiveness and dispersibility of these products. A method of manufacturing plate-shaped titanium oxide was disclosed in Japanese patent No.2979132 and this manufacturing method was for producing titanium oxide without consideration for luster or any interference color.

Incidentally, colors are initially a vital element, which has an impact on human being both physiologically and mentally. In fact, colors are a means for creating a safe and efficient work environment or a healthy and comfortable living environment. Colors can have physiological and psychological effects on people and therefore, color technology has actively been used in various fields.

In general, various types of color pigments are used for coloring substances. These color pigments produce desirable color shades by benefiting from phenomena such as absorption and the scattering of light, but the engineering of applying color pigments only simply does not meet today's diverse color sensitivity and design requirements. Subsequently, in addition to these color pigments, pearl luster pigments have been introduced, which are pigments made of titanium dioxide-coated mica that benefit from the interference phenomenon of light,.

The major characteristic of the pearl luster pigment is that it can provide the "flip-flop effect", which changes its color shade subtly by a slight change in angle.

This pearl luster pigment is widely used in many different fields; for cosmetics, paints, adhesives, printing inks, resin pastes etc. However, it has the following disadvantages; the pearl luster pigment consists of thin flakes or plate-shaped particles of natural mica, synthetic mica, alumina, silica, silicate glass or boric glass as its core component. These particles are coated in titanium dioxide and depending on the amount of coating, the particles create different pearl-like luster effects with various interference colors. Therefore, when such a pearl luster pigment is added to cosmetics, paints, printing inks, adhesives and resin pastes as an externally-used ingredient, the film thickness of the particles cannot be regulated because the particle thickness is far too great to form a super-thin film, and it also deters the design effect due to the lack of "flip-flop effect" since the scattering of light on the particle edges is too intense to create such a effect.

On the subject of composing plate-shaped titanium oxide particles, as Japanese patent No.2979132 describes, thin flake-shaped titanium oxide, which has a porous form with large specific plane, is produced by bringing cesium titanate into contact with acid solution to create layered crystals, followed by heating and exfoliating the crystal layers. According to the abovementioned patent, when the titanium oxide crystal layers are separated by the single layer, the layer thickness is very thin at a very small nanometer level. However, each layer is so thin that the thin flake-shaped titanium oxide cannot obtain any interference color.

Meanwhile, as described in Japanese patents No. 62-23793, 62-247834 and 62-213833, plate-shaped titanium oxide is produced by heating sol-gel of titanium alcoholate, which is attached to a drum, to oxidize it, followed by separating it from the drum using a scraper, preceding a sintering process to form a slightly pearl-like shape plate. Because the above separation is performed using a scraper, the particles are in a curly shape and the particle thickness is of micron order, thus, they cannot produce any interference color.

### Description of the invention

This present invention was intended to bring a solution to the aforesaid problems and new and outstanding luster as well as color brightness has been developed as a result within a recognized color range by interference colors. Also, the invention has successfully been completed with a discovery that the blending of a highly iridescent titanium oxide composition, which retains unclouded complementary colors, in cosmetic formulas provides not only a smooth texture to the skin and natural transparency but also control of pigmentation irregularities (blemish control effect) plus excellent anti-UV and photochromic effects.

In other words, as a result of a devoted, strongly committed and through study with the aim of solving the abovementioned problems, the invention presents a highly iridescent titanium oxide composition newly produced by the exfoliation of layers of titanium coating composition formed on the surface of thin flake-shaped matrix during the manufacturing process.

More specifically, the surface of the thin flake-shaped matrix is coated in layers of metal oxide and/or metal hydroxide, which is based on anatase or rutile type titanium oxide, and a new exfoliated composition is acquired by separating the layers of the coating composition by applying an alkali compound. This exfoliated composition is produced as a composition which retains the desired degree of luster as well as creating an interference color.

That is to say, the composition, to which the present invention pertains, consists of a thin flake-shaped matrix of one or two kinds or more that are selected from natural mica, synthetic mica, glass flake, silica flake, alumina flake and barium sulfate, plus a titanium oxide composition and/or titanium hydroxide composition, which coats the matrix surface. Furthermore, the exfoliated composition, which is a highly iridescent titanium oxide composition, is a titanium composition that is exfoliated from the above coating composition.

The aforesaid exfoliated composition is a highly iridescent titanium oxide composition and it has a luster degree of 55 - 90 in 60°, when measured using Gloss Checker IG-330 manufactured by Horiba International Corporation, when one part of the exfoliated composition is mixed with thirty parts of clear acrylic lacquer and the mixture is applied onto the black side of a black-and-white hiding chart JISK5400 using a 4-mil applicator. The composition produces colors by the interferential effect of light.

### Brief descriptions of the drawings

The following describe the best example for carrying out the present invention with reference to the drawings: Fig.1 is a conceptual diagram of the coating composition related to the highly iridescent titanium oxide composition according to the invention. Fig.2 is a schematic diagram showing a typical manufacturing method of the highly iridescent titanium oxide composition according to the invention. Fig.3 is a graph showing the results of the X-ray diffraction analysis of the coating composition according to the invention. Fig.4 is a micrograph image of the titanium oxide surface of the coating composition according to the invention. Fig.5 is a table showing the relationship between each interference color of the titanium oxide composition, i.e. the exfoliated composition according to the invention, and the thickness of the titanium layer. Fig.6 and Fig.7 display micrograph images of the titanium oxide composition, i.e. the exfoliated composition. Fig.8 is a graph showing the results of the analysis implemented on the titanium oxide composition, i.e. the exfoliated composition according to the invention, using a 3-D glossmeter.

### Best example for carrying out the invention

The coating composition, from which the highly iridescent titanium oxide composition is acquired, the highly iridescent titanium oxide composition, and the manufacturing methods of these compositions, to all of which the present invention pertains, are explained in this specification as the coating composition, which forms layers consisting of titanium oxide or titanium hydroxide on the surface of a thin flake-shaped matrix, the exfoliated composition, which is acquired by exfoliating the layers of the coating composition and the manufacturing methods of these compositions. The significant improvement of the prior art is that the exfoliated composition can be used for various purposes under specific conditions and when it is contained in a cosmetic formula, for instance, a layer containing the exfoliated composition is formed on the skin to create highly iridized coloration benefiting from the interferential effect of light, which was rare in the prior art.

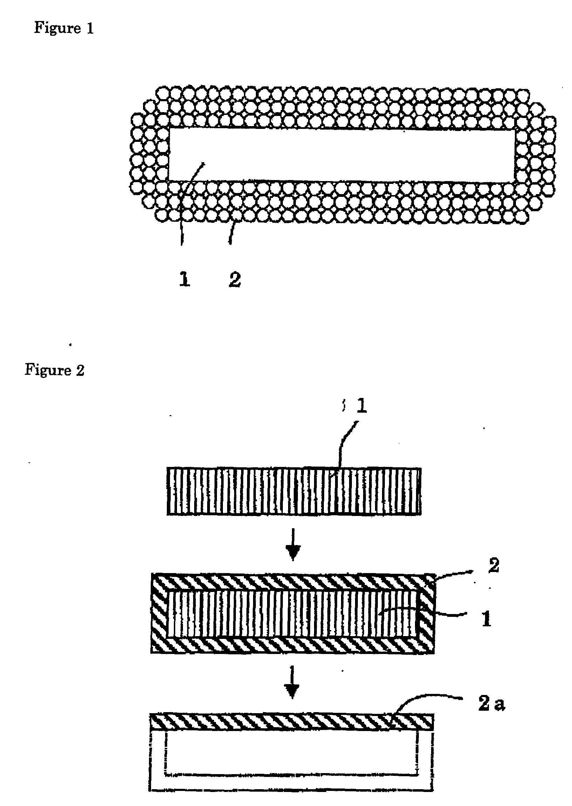

As illustrated in Fig.1, the coating composition, from which the highly iridescent titanium oxide composition is acquired and to which the present invention pertains, is a composition whose coating layers (2) consist of metal oxide and/or metal hydroxide based on anatase or rutile type titanium oxide. These layers form on the surface of thin flake-shaped matrix (1), providing a certain thickness between 0.05µm and 0.6µm to produce luster.

The optimum particle diameter for the matrix is 100 - 700µm and using a matrix with a particle diameter of less than 100µm prevents an interference color from achieving high iridization because the particles of exfoliated composition are too fine, which will be explained in detail later. On the other hand, when the particle diameter of matrix becomes greater than 700µm, it causes an undesirable roughness to the particle of exfoliated composition though it enhances an interference color with more intense iridization. In addition, a particle diameter between 100µm and 300µm intensifies luster even more as well as enabling easy exfoliation.

More precisely, substances ideal for providing thin flake-shaped matrix (1) with a particle diameter that falls within the aforesaid range satisfactorily are natural mica, synthetic mica, glass flake, silica flake, alumina flake and barium sulfate. Especially, silica flake is a desirable choice for thin flake-shaped matrix (1), as it is relatively easy to control its form and surface smoothness as well as enabling fairly even application of coating layers (2) to the surface, providing a certain thickness to create luster.

There is no particle thickness specified for thin flake-shaped matrix (1) but the range between 0.1µm and 10µm is considered to be ideal. When the particle is less than 0.1µm thick, the matrix's outer edge curls up and prevents obtaining full interference luster of titanium oxide or titanium hydroxide coating. Alternatively, a particle thickness of more than 10µm causes confliction between the interference color produced by titanium oxide or titanium hydroxide layers that are coated over the matrix particle in the plane and thickness directions, and the interference color created by titanium oxide or titanium hydroxide layers that are exfoliated from the matrix particle in the plane and thickness directions. Consequently, the interference color confliction prevents full luster.

Coating layer (2) is a layer that consists of a titanium oxide and/or titanium hydroxide composition and is coated over a matrix to a specific thickness to create luster. Such a composition is ideally produced from titanyl sulfate or titanium tetrachloride solution or alternatively by hydrolysis of titanium alcoholate. Also, a reinforcer such as silica, alumina, Zr, Ce, and/or Zn can be added to coating layer (2) in order to improve its resistance against light and the indestructibility of exfoliated composition.

A group of particles, the size of which varies within the range from 0.01µm to 0.05µm or preferably from 0.02µm to 0.04µm, forms the coating composition and the exfoliation operation performs best when the particle size of matrix (1) is 300µm and the particle size of the titanium oxide or titanium hydroxide composition is 0.02µm. When the particle size of matrix (1) is 800µm, the exfoliation of coating layer (2) is still practicable with a particle size of 0.01µm. However, if the particle diameter of matrix (1) becomes less than 100µm, the exfoliation operation is no longer feasible unless the particle size of coating layer (2) is 0.05µm. In other words, the particle diameter of matrix (1) and the particle diameter of coating layer (2) are in inverse proportion to one another.

According to the present invention, the coating composition illustrated in Fig. 1 is formed first and coating layer (2) is exfoliated from the coating composition next, as described in Fig.2, by which exfoliated composition (2a) is acquired. That is to say, the highly iridescent titanium oxide composition is exfoliated composition (2a), which is produced by exfoliating metal oxide or metal hydroxide, i.e. coating layer (2) of the coating composition, from the surface of thin flake-shaped matrix (1).

Some commercially available thin and fine flake-shaped particles may be used for thin flake-shaped matrix (1), but the usual choices are natural mica, synthetic mica, barium sulfate, silica flake and alumina flake. One of the methods to produce a matrix, for instance, is to sinter chip-shaped natural mica of Indian origin at a temperature of 800°C for two hours followed by soaking the sintered natural mica chip in clean water for five days. The natural mica chip is then pulverized using a masscolloider and the particles are graded to acquire a matrix.

Also, further coating can be applied to the matrix surface before exfoliation with a silica or alumina compound, which consists of water glass, organic silica or soluble aluminum salt. Extra coating such as the above enables production of non-curling exfoliated composition with good weather resistance.

As described above, the coating composition is a titanium oxide or titanium hydroxide composition, which is first applied to the surface of thin flake-shaped matrix (1) to form coating layer (2) to a specific thickness in order to acquire luster. The coating composition is ideally produced from titanyl sulfate or titanium tetrachloride solution or alternatively by hydrolysis of titanium alcoholate.

Secondly, prior to acquiring the exfoliated composition, the coating composition is sintered at a temperature between 300°C and 800°C and then its coating layer is exfoliated from the thin flake-shaped matrix in alkaline solution (pH8 or greater). This process enables production of non-curling smooth exfoliated composition.

Subsequently, the luster and interference colors of the exfoliated composition are also enhanced. The particles of the exfoliated composition curl up at a sintering temperature below 300°C, whereas a temperature higher than 800°C condenses titanium oxide or titanium hydroxide in the exfoliated composition to produce poor luster and interference colors after exfoliation.

The exfoliated composition is sintered at a temperature between 500°C and 900°C in order to prevent curling after it is filtered, cleaned with water and dried. Another post-treatment for the exfoliated composition is coating it with iron oxide, cobalt, nickel, lithium, sodium, potassium or a colored inorganic compound. Coloring the exfoliated composition by coating it with an organic pigment is also a feasible treatment under one of the known methods depending on the intended purpose and the mode of implementing the method.

Therefore, the highly iridescent titanium oxide composition according to the present invention is the exfoliated composition acquired by the procedures explained above, and the thickness of its particles is specified between 0.05µm and 0.6µm. This implies, in terms of the relationship between two aspects of film thickness; geometrical and optical (film thickness × refractive index) for producing rainbow colors, that a geometrical thickness of 0.05 - 0.5µm and an optical thickness of 0.1 - 0.9 µm are desirable.

Designing the composition too thinly makes it more difficult to produce the required interference color, whereas the composition with excessive thickness decreases the intensity of interference color due to the scattering thickness, thus, it is not desirable.

Shown in Table 1(Fig.5??) is the relationship between the interference color and the geometrical thickness of the titanium composition. The highly iridescent titanium oxide composition according to the invention was applied onto a piece of tape and the tape was placed against a black background for the observation.

The preciseness in making equal-sized particles of the highly iridescent titanium oxide composition according to the invention increases the saturation of rainbow colors. In other words, it enables the creation of required interference color by the scattering of light on each particle of the thin flake-shaped highly iridescent titanium oxide composition.

According to the present invention, the diameter and thin flake form of the particles of the highly iridescent titanium oxide composition are consistent. In a more precise sense, providing that the average particle diameter for a scattering length by laser diffraction is Aµm, a volume distribution of more than 60% or preferably more than 70% obtaining the particle diameter within A±4µm provides an ideal condition. Also, when counting the number of thin flake-shaped particles which are magnified 2000 times using a scanning electron microscope, it is desirable that less than 10% of the total form a flake shape, whose tangential line exceeds its thickness more than 1.5 times.

In order to maintain such consistency of particle diameter (and form), careful pulverization and particle grading play a vital role in controlling the smoothness of the thin flake-shaped matrix. When selecting natural mica for the matrix, for instance, its high consistency should be ensured by the agitation and pulverization of ore of 2 - 8 mesh by wet process preceding the particle grading by elutriation.

Furthermore, even coating of the titanium composition on the smooth surface of the thin flake-shaped matrix, which consists of uniform-size particles, preceding the exfoliation of the titanium composition guarantees production of homogeneous interference colors.

The highly iridescent titanium oxide composition according to the invention, i.e. the exfoliated composition, which is produced as above, is therefore capable of producing interference colors, as a result of allowing the coating composition to form a layer of a specific thickness in order to create luster. Subsequently, when the exfoliated composition is used in paints for instance, light, which is directly reflected from a surface, and incident light, which is reflected when transmitted through the surface, interfere with each other to cause a phase-mismatch; thus, highly iridescent interference colors are produced.

When applying the highly iridescent titanium oxide composition according to the invention onto a surface to form an even layer, light which is reflected from the particle surface of the applied area, and light which is transmitted through the titanium composition particles to be reflected by another medium of different refractive index, interfere with each other to produce a particular type of interference color. The type of interference color as such is determined by the thickness of the coating composition layer.

### (Comparative example 1)

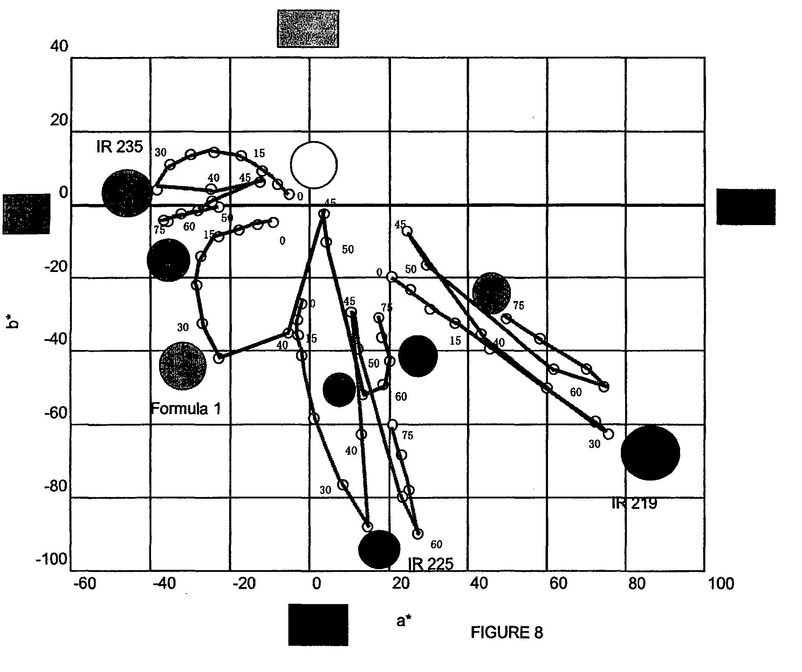

Fig.8 is a graph showing the results of the analysis of the titanium oxide composition, i.e. the exfoliated composition according to the invention, using a 3-D glossmeter.

One part of the exfoliated composition according to the invention was mixed with thirty parts of clear acrylic lacquer and the mixture was applied onto the black side of a black-and-white hiding chart JISK5400 using a 4-mil applicator. Using 3D Glossmeter GCMS-4, a color profile of the mixture was taken in order to apply the Lab conversion (?) at an incident angle of 45° and a reflection angle (measuring angle) between 0° and 75° fluctuated by every 5°.

Pearl luster pigments manufactured by Merck Ltd. Japan; Iriodin ("IR" hereafter) 225, 219 and 235, were used for comparison. It became clear by the analysis results that the titanium oxide composition according to the invention produced a wider range of interference colors.

Detailed below are examples of manufacturing methods of the titanium oxide composition, i.e. the exfoliated composition according to the invention. The technical scope of the invention is not restricted by these manufacturing methods.

### (Manufacturing method 1)

1.0kg of chip-shaped natural mica of Indian origin was sintered in the atmosphere at a temperature of 800°C for two hours. The sintered chip-shaped natural mica was left to cool down and was soaked in 10 ℓ of clean water at room temperature for five days. The sintered chip-shaped natural mica was then put through a 500µm masscolloider manufactured by Masuko Sangyo Co., Ltd. to be pulverized twice. After the pulverization, the sintered natural mica powder was transferred into a 50ℓ plastic canteen and 0.02% hexametaphosphate solution was added to make up a total volume of 45ℓ.
The sintered natural mica powder and the hexametaphosphate solution were combined thoroughly using a propeller mixer and when the mixture set after five minutes, the clear supernatant liquid was removed and placed into a separate container. This process was repeated three times. Large particles of more than 0.1mm diameter were separated and the clear supernatant liquid was also passed through a 10 mesh sieve (standard size of 800µm) and a 65 mesh (203µm) sieve. 150g of 10 - 65 mesh particles were collected as a result.

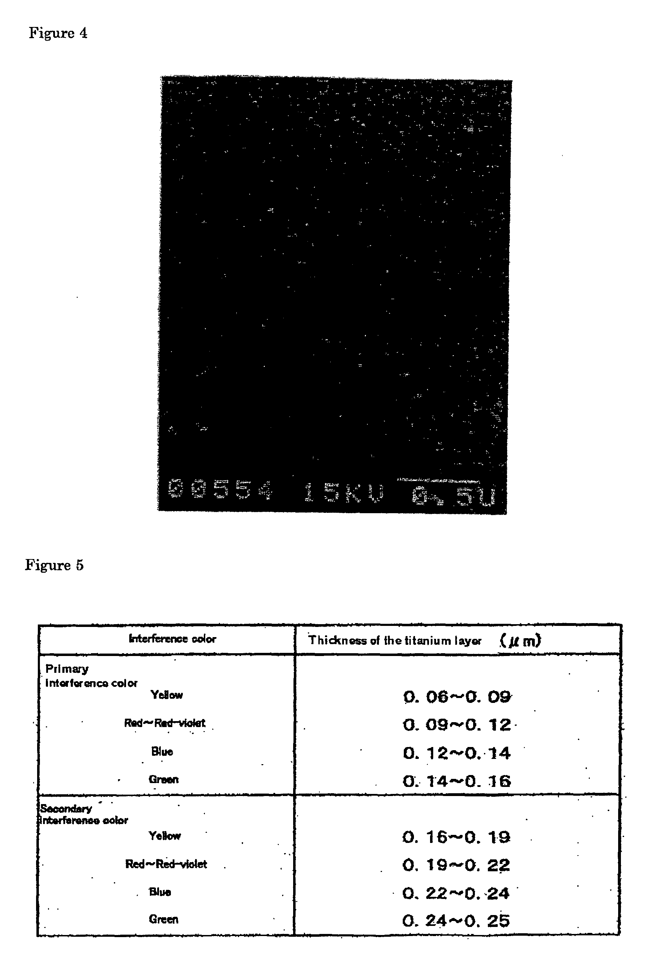

Next, 400g of titanyl sulfate and 7.5ℓ of clean water were added to the 150g mica and they were combined using the propeller mixer until the titanyl sulfate was thoroughly dissolved. The mixture was then heated, while being stirred, at a temperature of 90°C or over for four hours to be hydrolyzed. The hydrolysate was left to cool down and then was cleaned with water, filtered and dried at 150°C as well as sintered at 300°C for two hours. A small section was removed from the surface for SEM observation, when multiplied 20,000 times, using Hitachi S-2100B electronic microscope (SEM). As the result shown in Fig.4, the coating composition was formed by a group of particles of 0.02µm diameter.

Also, 10% caustic soda solution was added to the water cleaned powder to adjust the pH level to 11 and the powder was left, soaked in the solution, until it set. Later, the supernatant powder was separated by decantation, filtered and cleaned with water. The cleaned powder was combined with 5ℓ of water solution containing 8.325g of aluminum sulfate, plus 4g of zirconium oxychloride and 18g of urea were also added to the powder. The mixture was heated, while thoroughly stirred by propeller mixing, at a temperature of 80°C or over for five hours until hydrolyzed. The hydrolyzed powder was left to cool down, cleaned with water, filtered, dried at 150°C and then sintered in the atmosphere at 700°C for two hours. 140g of powder was acquired as a result.

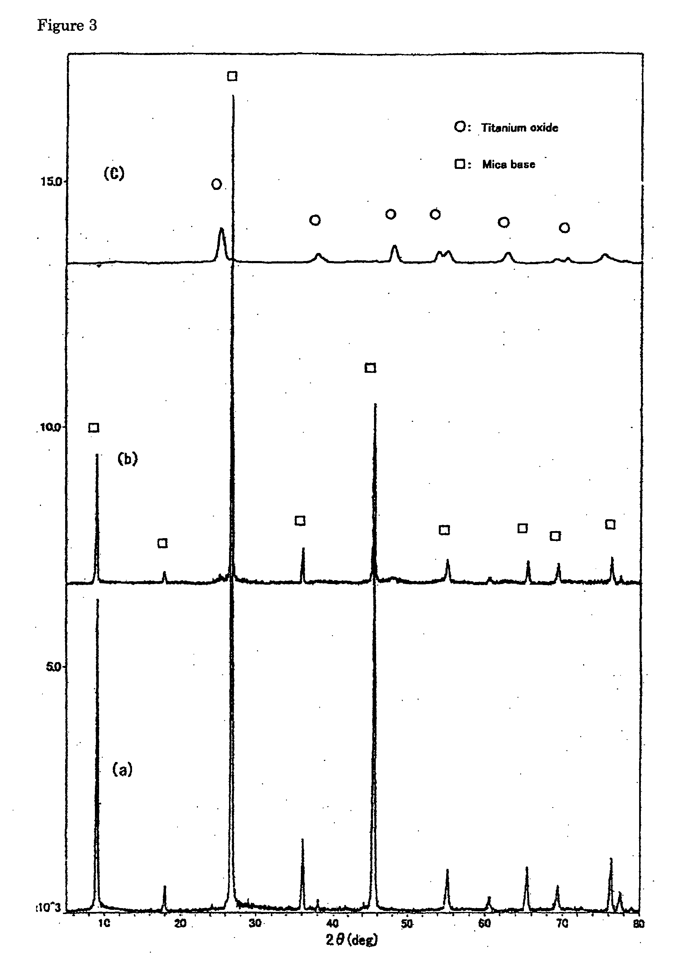

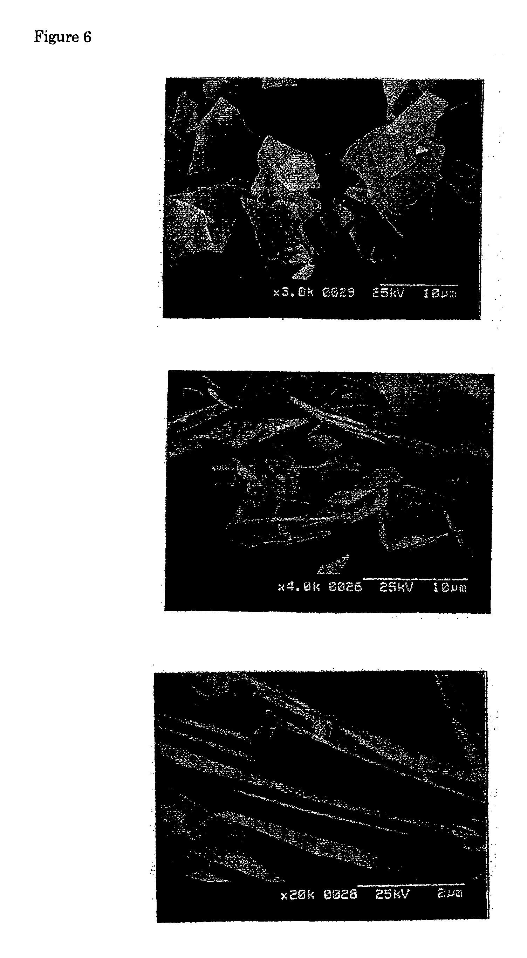

The powder was measured using Rigaku's X-ray diffractometer MiniFlex. Diffraction patterns were recorded and pattern (C) in Fig.3 indicated that the powder consisted of anatase titanium oxide, though it was a very broad type. In addition, as the results of scanning electron microscope (SEM) show in Fig.6, the average plate diameter of the thin flake-shaped particle was measured to be 10µm and the thickness was 0.24µm when magnified both 2,000 times and 20,000 times. Also, a luster degree of 85 at 60° was measured, using Gloss Checker IG-330 manufactured by Horiba International Corporation, when the mixture of 1g of the above powder and thirty parts of clear acrylic lacquer was applied onto the black side of a black-and-white hiding chart JISK5400 using a 4-mil applicator.

The color dependency against a black background was also examined using 3D Glossmeter GCMS-4 manufactured by Murakami Research Laboratory. A color profile of the above mixture was taken at an incident angle of 45° and at a reflection angle (measuring angle) between 0° and 75° varied by every 5°. The color profile was then converted to a hue by the L.a.b conversion to be plotted in a graph along a. and b. As the results are shown in Fig.8, a wide range of color profiles was recorded varying from green to turquoise and changing further to blue.

Furthermore, in order to examine the composition of the powder, it was weighed exactly to be 0.2g, heated and dissolved together with sulfuric acid and aluminum sulfate. Water and hydrochloric acid were added to the cool mixture and aluminum was added to proceed with the reduction of titanium. When the mixture cooled down, the amount of titanium oxide (%) was measured by titration using aluminum ferric sulfate (III) solution with potassium thiocyanate solution as an indicator. As a result, the proportion of titanium oxide was measured to be 95.0%. Meanwhile, the quantities of aluminum and zirconium were determined by analytical method based on the reference peak of 394.40nm for aluminum and the reference peak of 343.82nm for zirconium, which were rated using Rigaku's wavelength dispersive X-Ray fluorescence spectrometer ZSX100e. According to the result, 1.15 % aluminum was identified in form of alumina, and zirconium was measured to be 1.08% in form of zirconium oxide. This means that the powder was recognized as a composition which consisted of 95.0% titanium oxide, 1.15% alumina and 1.08% zirconium oxide.

### (Manufacturing method 2)

10kg of synthetic mica (by Topy Industries, Ltd.), the particles of which were of 150 - 650µm laser diameter with an average diameter of 300µm, was placed in a 600ℓ glass-lined tank with a jacket, and 400ℓ of clean water, 175g of stannic chloride and 1 mol/ℓ of aqueous sulfuric acid solution were added to the tank to adjust the pH level of the mixture to 1.9. The mixture was heated, while stirred, and when the temperature reached 80°C, it underwent a dripping process for 10 hours to stimulate chemical reactions using 1/3 mol/ℓ of titanium tetrachloride hydrochloric acid solution and 15% caustic soda solution for obtaining the above pH level at 0.12kg/minute flow speed of the titanium tetrachloride hydrochloric acid solution.

The mixture was left to cool down and after stirring was stopped, the supernatant was separated by decantation. The remaining powder in the mixture was stirred again with 400ℓ of clean water, and 15% caustic soda solution was also added to obtain pH6.5 - 7.5. The mixture was then filtered, cleaned with water and dried at a temperature of 150°. The dried powder was also sintered in the atmosphere at 800°C for two hours. The particle surface was examined by SEM observation as in manufacturing method 1 and it was confirmed that the powder was formed by a group of particles of 0.016µm.

The sintered powder was soaked in 400ℓ of sodium carbonate solution at pH12 for five days, after which the mixture was stirred to disperse the powder thoroughly. The mixture was stirred further with 2ℓ of 2% hexameric phosphate soda solution. Once the powder was well dispersed in the mixture, the supernatant powder was separated by decantation. This process was repeated until all the supernatant powder was collected.

The powder in the supernatant liquid was cleaned with water and filtered. The filtered powder was then soaked in 400ℓ of caustic soda solution at pH9.2 and the solution mixture was stirred thoroughly to disperse the powder. Obtaining the pH level of 9.2, the mixture was heated up to 80°C and 3mol/ℓ. of water glass solution and 6N hydrochloric acid solution were gradually added to the mixture over a period of two constant hours at a flow rate of the water glass solution of 0.085/minute. The mixture was aged for another two hours and was left to cool. It was then filtered, cleaned with water and dried at 150°C. 18kg of powder was acquired as a result.

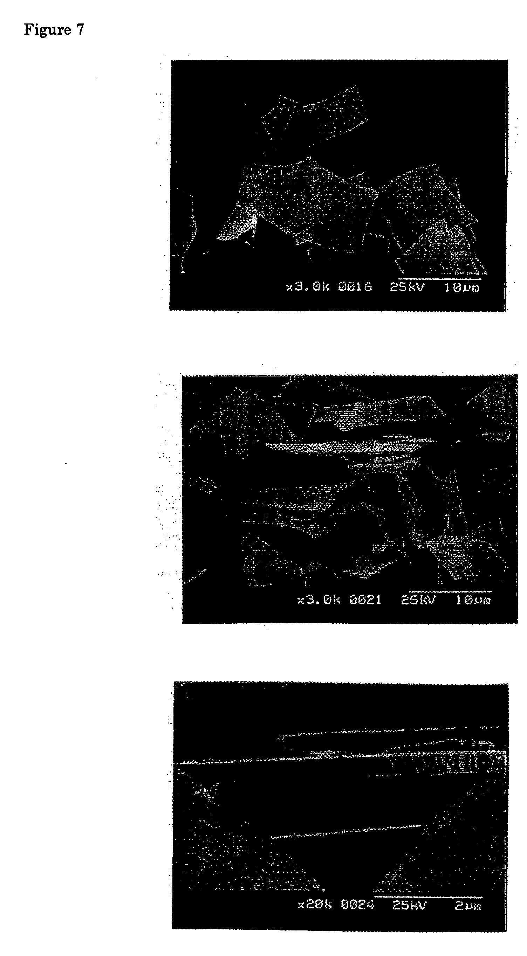

X-ray diffraction measurement was carried out using the same device as in manufacturing method 1, the result of which indicated that the powder consisted of rutile titanium oxide. Also, it was identified by SEM observation that each particle formed a thin flake shape whose average dimension was a flake diameter of 15µm and a flake thickness of 0.45µm, as shown in Fig.7.

As in manufacturing method 1, the powder color was examined using a black-and-white hiding chart JISK5400 and a 3D glossmeter. Meanwhile, the powder composition was also analyzed. Indicated by the results, the gloss value was 80 at an angle of 60, and for the color dependency, a wide range of color profiles was recognized varying from yellow green to green and changing further to turquoise. Meanwhile, the result of composition analysis revealed that the powder consisted of 88.5% titanium oxide and 10.3% silica.

### (Manufacturing method 3)

1kg of glass flakes (supplied by Nippon Sheet Glass Co., Ltd.), the particles of which were of 50 - 200µm laser diameter with the average diameter of 140µm, were placed in a 40ℓ enamel container with 17ℓ of 10% titanyl sulfate solution, 5ℓ of 0.06mol/ℓ aqueous sulfuric acid solution, 100g of urea and 10ℓ of clean water. The mixture was heated up to over 90°C, while being stirred using a propeller mixer, to undergo a six-hour hydrolysis process.

After it was left to cool down, the mixture was separated by decantation repeatedly and subsequently cleaned with water. 10% caustic soda and 30ℓ of clean water solution was added to the mixture in order to stabilize the pH level at 8.8 and the mixture was heated up to 80°C. 0.85ℓ of 1mol/ℓ glass water solution and 1N hydrochloric acid solution were gradually added to the mixture for one hour at a flow rate of the water glass solution of 0.014ℓ/minute, while the pH and temperature obtained were stable. The mixture was then aged for three hours.

Once the mixture was left to cool down, the particle surface was examined by SEM observation, as in manufacturing method 1, by which a large particle size of 0.04µm was identified.

1mol/1 sodium carbonate was added to the aged dispersion liquid to adjust the pH level to 12 and the liquid was left undisturbed for four days. The mixture was then stirred again using the propeller mixer and was left undisturbed again for a while after which the powder in the supernatant liquid was separated by means of decantation. This process was repeated until all the powder in the supernatant liquid was collected. The collected powder was then cleaned with water, filtered and dried at a temperature of 150°C. Later, the dried powder was sintered in the atmosphere at 300°C for one hour, and finally, 650g of powder was acquired.

According to the X-ray diffraction result, the acquired powder consisted of a very broad type of anatase. Meanwhile, thin flake forming particles with the average dimension of 8µm flake diameter and a thickness of 0.35µm were identified by the SEM observation.

The properties of the powder were also examined using the means as in manufacturing method 1 and according to the results, a gloss value of 60 at an angle of 60° as well as the color dependency with a wide range of color profiles varying from burgundy to red and yellow were indicated. The composition analysis also revealed that the powder consisted of 70% titanium oxide and 2.8% silica with the remaining ingredient being water.

### (Manufacturing method 4)

Chip-shaped natural mica of Indian origin was pulverized and the particles were graded by the same means as in manufacturing method 1. 1kg of the mica was placed in a 40ℓ enamel container with 17ℓ of 10% titanyl sulfate, 5ℓ of 0.06mol/ℓ aqueous sulfuric acid, 100g of urea and 10ℓ of clean water. The mixture was heated to over 90°C, while being stirred using a propeller mixer, to undergo a six-hour hydrolysis process.

After it was left to cool down, the mixture was cleaned with water and filtered. 10% caustic soda solution and 30ℓ of clean water were added to the mixture to adjust the pH level to 10 and the mixture was heated to 80°C, while being stirred with nitrogen gas.
While obtaining the pH level and temperature, 0.2ℓ of 0.5mol/ℓ ferrous sulfate solution and 10% caustic soda solution were gradually added to the mixture for 15 minutes at a flow rate of the ferrous sulfate solution of 0.014ℓ/minute. The mixture was stirred for another five hours with air this time instead of nitrogen gas. When the mixture cooled down, the surface was examined by SEM observation as in manufacturing method 1 and particles of 0.04µm were identified with particles of 0.3µm scattered on their surface.

1mol/ℓ sodium carbonate solution was added to the aged dispersion liquid to adjust the pH level to 12 and the liquid was left undisturbed for four days. The mixture was then stirred again using the propeller mixer and was left undisturbed again for a while after which the powder in the supernatant liquid was separated by means of decantation. This process was repeated until all the powder in the supernatant liquid was collected. The collected powder was then cleaned with water, filtered and dried at a temperature of 150°C. Later, the dried powder was sintered in the atmosphere at 500°C for one hour and 650g of sintered powder was acquired as a result.

It was identified according to the X-ray diffraction result that the acquired powder consisted of anatase and iron oxide (Fe203). The SEM observation result also indicated that each particle formed a thin flake shape with the average dimension of 8µm plate diameter and a thickness of 0.35µm.

The properties of the powder were also examined using the means as in manufacturing method 1 and according to the results, a gloss value of 60 at an angle of 60° as well as the color dependency with a wide range of color profiles varying from burgundy to red and to yellow were indicated. The composition analysis also revealed that the powder consisted of 99% titanium oxide and 1% iron oxide.

The exfoliated composition according to the invention, which can be manufactured using different methods as described above, is a composition that may be used for cosmetic purposes. The ideal proportion of the exfoliated composition in a cosmetic formula is 0.5 - 50% by weight. A proportion falling below 0.5 will not induce the effect of controlling pigmentation irregularities sufficiently, whereas a proportion higher than 50% will over-intensify the iridization, which is undesirable.

Cosmetic formulas according to the present invention may be used in cosmetic skin products such as skincares, under makeups, sunscreens, cream foundations, powder foundations, pressed powders, eyeliners, cheek colors, lipsticks, nail enamels etc. The finished dosage form is not particularly determined and these cosmetic products may be produced under typical manufacturing methods unless the exfoliated composition according to the invention is blended in their formulas.

As well as the aforesaid exfoliated composition, the cosmetic formulas according to the present invention may contain ingredients that are typically blended in various cosmetic products, such as powder-formed ingredients (pigments, dyes and resins), oils, surfactants, fluorine compounds, resins, alcohols, high-molecular compounds, UV protection agents, antioxidants, gums, preservatives, fragrances, moisturizers, physiologically active components, salts, solvents, chelating agents, neutralizing agents, pH adjusters, water and others. An adequate amount of each ingredient can be combined according to the intended use, purpose, dosage form etc.

Powder-formed ingredients typically used in cosmetics include dyes such as red 104, red 201, yellow 04, blue 01 and black 401; lake pigments such as yellow 04A1 lake and yellow 203Ba lake; high polymers such as nylon powder, silk powder, urethane powder, Teflon powder, silicon powder, cellulose powder, silicon elastomer, chitin, chitosan and calcium alginate; color pigments including yellow iron oxide, red iron oxide, black iron oxide, chromium oxide, carbon black, ultramarine blue pigment and royal blue pigment; white pigments such as titanium oxide, zinc oxide and cerium oxide; extenders including talc, mica, sericite, kaolin, barium sulfate, aluminum oxide, silicon dioxide, calcium carbonate, magnesium carbonate, aluminum silicate and magnesium silicate; UV protection powders such as fine-particle titanium oxide, fine-particle zinc oxide and fine-particle cerium oxide; pearl pigments like mica-titanium; bentonite; and smectite. The form and size of these powders are not particularly determined.

It also does not matter whether or not the abovementioned powders are treated by widely known surface finishing methods using silicon, silane, fluorine compound, metal soap, wax, fatty acid, N-acylated lysine, water-soluble polymer compound, resin or plasma, or even mechanochemically, for instance.

Oils normally applied are; liquid oils such as liquid paraffin, squalane oil, cetyl 2-ethyl hexanoate, isopropyl myristate, olive oil and caster oil; semisolid to solid oils including petrolatum, solid paraffin, beef tallow oil, lanolin, beeswax, spermaceti wax and cholesterol; higher alcohols such as cetanol and behenyl alcohol; higher fatty acids such as palmitic acid and stearic acid; fluorine-based oleums such as perfluoropolyether; silicon-based oleums; and silicon derivatives.

Surfactants commonly employed are; nonionic surfactants including sorbitan fatty acid ester, glycerin fatty acid ester, polyoxyethylene ("POE" hereafter) sorbitan fatty acid ester, POE glycerin fatty acid ester, POE alkyl ether, POE polyoxypropylene alkyl ether, POE polyoxypropylene copolymer, POE alkyl phenyl ether, POE hydrogenated caster oil, polyethylene glycol fatty acid ester, decaglycerin fatty acid ester and alkyl diethanolamide; anionic surfactants such as alkyl sulfate, POE alkyl ether sulfate, POE alkyl ether acetate, alkyl phosphate, POE alkyl ether phosphate, higher fatty acid salt, higher fatty acid hydrolyzed collagen salt, amino acid based anionic surfactant, sulfosuccinate surfactant and olefin sulfonic acid salt; amphoteric surfactants such as of lecithin and those based on betaine acetate and imidazolinium betaine; and cationic surfactants such as alkyl trimethyl ammonium chloride, dialkyl dimethyl ammonium chloride and alkyl dimethyl benzyl ammonium chloride.

Alcohols typically used are monohydric alcohols such as ethanol, propanol, benzyl alcohol etc. and polyvalent alcohols including 1, 3-butylene glycol, propylene glycol, dipropylene glycol, glycerine, polyethylene glycol, sorbitol etc. As for high-molecular compounds, polyvinylpyrrolidone, vinylpyrrolidone vinyl acetate copolymer, acrylic resin alkanolamine, vinyl acetate crotonic acid copolymer, methyl vinyl ether-maleic acid monoalkyl ester copolymer, N-methacryloyl ethyl-N, N-dimethyl ammonium-α-N-methyl carboxy betaine/methacrylic acid alkylester copolymer, diethyl sulfate vinylpyrrolidone-N, N-dimethylamino ethyl methacrylate copolymer, hydroxyethyl cellulose dimethyl diallyl ammonium chloride, hydroxyethyl cellulose hydroxypropyl trimethyl ammonium chloride ether, carboxyvinyl polymer, sodium carboxy methyl cellulose, hydroxyethyl cellulose, xanthan gum etc. are widely used ingredients.

Other ingredients, which are as typically used as the above, are moisturizers such as amino acid and hyaluronic acid; UV absorbents such as oxybenzone and para-aminobenzoate ester; antioxidants such as tocopherol, dibutyl and hydroxytoluene; preservatives such as paraben and phenoxyethanol; bactericides including isopropyl methyl phenol and triclocarban; protein hydrolysates including collagen, keratin, silk etc.; pH adjusters such as citric acid and sodium citrate; anti-inflammatory agents such as plant extracts and dipotassium glycyrrhizinate; inorganic salts such as sodium chloride etc.; chelating agents; dyes; and fragrances.

Described below are typical formula examples. The technical scope of the invention is not restricted by these formulas. The proportion of each cosmetic ingredient is indicated by % by mass unless otherwise stated. The details of the sensory test, which was used for the invention, are also stated prior to the descriptions of the formula examples.

Sensory testing method: a panel of twenty specialists was invited to try out each cosmetic formula to evaluate its effects in terms of smoothness to the skin, natural transparency and pigmentation irregularity control (texture, blemishes etc.) First, the evaluation result of each formula was given by each assessor on the panel based on the set of criteria shown below and then a total score for each formula was calculated.

### (Assessment grades and scores)

5 points: Very good
4 points: Good
3 points: Satisfactory
2 points: Poor
1 point: Very poor

### (Total scores and symbols)

ⓞ : The total score is 80 points and over.
○: The total score is over 60 points but below 80 points.
Δ: The total score is over 40 points but below 60 points.
×: The total score is 40 points and under.

For prescribing formulas 1 and 2, and comparative formulas 1 and 2, the ingredients 1 - 12 listed in table 1 were thoroughly stirred together, to which the ingredients 13 - 20 that were evenly heated and mixed together were added. The mixture was then pulverized and pressed into a container. Formula 1 was formulated with the exfoliated composition, which was produced under manufacturing method 3 according to the present invention, whereas formula 2 contained the exfoliated composition, which was produced by manufacturing method 4.

**Table 1**

| | | Formula | | Comparative formula | |
|---|---|---|---|---|---|
| | Ingredient | 1 | 2 | 1 | 2 |
| 1 | Talc | 9 | 9 | 9 | 9 |
| 2 | Mica | 10 | 10 | 10 | 10 |
| 3 | Sericite | 18 | 18 | 18 | 18 |
| 4 | Synthetic mica | 15 | 15 | 15 | 15 |
| 5 | Exfoliated composition produced using manufacturing method 3 | 15 | | | |
| 6 | Exfoliated composition produced using manufacturing method 4 | | 15 | | |
| 7 | Barium sulfate | 5 | 5 | 5 | 5 |
| 8 | Titanium oxide | 12 | 12 | 12 | 12 |
| 9 | Iron oxide | 4 | 4 | 4 | 4 |
| 10 | titanium oxide-coated mica (red interference color) | | | 15 | |
| 11 | titanium oxide-coated mica (yellow interference color) | | | | 15 |
| 12 | Boron nitride | 2 | 2 | 2 | 2 |
| 13 | Petrolatum | 2 | 2 | 2 | 2 |
| 14 | Dimethylpolysiloxane | 3 | 3 | 3 | 3 |
| 15 | Liquid paraffin | 2 | 2 | 2 | 2 |
| 16 | Tri-iso-glyceryl octanoate | 2 | 2 | 2 | 2 |
| 17 | Glyceryl sesque-isostearate | 1 | 1 | 1 | 1 |
| 18 | Preservative | Adequate amount | Adequate amount | Adequate amount | Adequate amount |
| 19 | Antioxidant | Adequate amount | Adequate amount | Adequate amount | Adequate amount |
| 20 | Fragrance | Adequate amount | Adequate amount | Adequate amount | Adequate amount |

Displayed in table 2 are the results acquired from the sensory test implemented on formulas 1 and 2, and comparative formulas 1 and 2.

**Table 2**

| | Formula | | Comparative formula | |
|---|---|---|---|---|
| | 1 | 2 | 1 | 2 |
| Smoothness to the skin | ⓞ | ⓞ | ○ | ○ |
| Natural transparency | ⓞ | ⓞ | × | × |
| Pigmentation irregularity control by interferential action (blemish control effect) | ○ | ○ | ○ | ○ |
| Photochromic effect | × | ⓞ | × | × |
| ⓞindicates a high degree of photochromic effect and × indicates no photochromic effect. | | | | |

As shown in table 2, it was clarified that both formula 1 containing the exfoliated composition, which was produced under manufacturing method 3 according to the invention, and formula 2 also prescribed with the exfoliated composition, which was produced under manufacturing method 4, provided a smooth texture to the skin and natural transparency as well as the control of pigmentation irregularities (blemish control effect). It was also discovered that formula 2, in particular, was excellent in providing both anti-UV and photochromic effects.

Meanwhile, it became apparent that comparative formulas 1 and 2, which were formulated with titanium oxide-coated mica instead of the exfoliated composition, exhibited rather substandard quality in providing a smooth texture, natural transparency and the photochromic effect, which could not match the achievement that was reached with formulas 1 and 2 presented by the invention.

Listed below is a series of other formula examples that contain the exfoliated composition produced using the manufacturing methods according to the present invention. Incidentally, the same sensory test was implemented on these formulas and the outcome proved that they were as excellent as formulas 1 and 2.

### Formula 3 Compact powder foundation

| Ingredient | | Content (% by mass) |
|---|---|---|
| (1) | Silicon-treated sericite | 15 |
| (2) | Silicon-treated synthetic mica | 15 |
| (3) | Silicon-treated talc | Residual |
| (4) | Silicon-treated exfoliated composition produced using manufacturing method 2 according to the invention | 15 |
| (5) | Silicon-treated sphere silica | 5 |
| (6) | Boron nitride | 2 |
| (7) | Silicon-treated titanium oxide | 10 |
| (8) | Silicon-treated fine-particle titanium oxide | 7 |
| (9) | Silicon-treated iron oxide | 4 |
| (10) | Silicon-treated zinc oxide | 5 |
| (11) | Squalane oil | 3 |
| (12) | Dimethylpolysiloxane | 4 |
| (13) | Methylphenylpolysiloxane | 3 |
| (14) | Octylmethoxycinnamate | 2 |
| (15) | Sorbitan sesque-isostearate | 1 |
| (16) | Paraben | Adequate amount |
| (17) | Antioxidant | Adequate amount |
| (18) | Fragrance | Adequate amount |
| Ingredients 1 - 10 were mixed and pulverized first, and then the mixture of ingredients 11 - 18 were stirred in. The entire mixture was then pulverized and set in a container to form the compact powder foundation. | | |

### Formula 4 Oil-in-water type cosmetic

| Ingredient | | Content (% by mass) |
|---|---|---|
| (1) | Purified water | Residual |
| (2) | Propylene glycol | 4 |
| (3) | Glycerin | 2 |
| (4) | Sodium metaphosphate | Adequate amount |
| (5) | Bentonite | 1.5 |
| (6) | Potassium hydroxide | 0.5 |
| (7) | Palmitic acid | 1.1 |
| (8) | Isostearic acid | 1 |
| (9) | Titanium oxide | 10 |
| (10) | Iron oxide | Adequate amount |
| (11) | Exfoliated composition produced using manufacturing method 4 according to the invention | 10 |
| (12) | titanium oxide-coated plate-shaped barium sulfate | 3 |
| (13) | Talc | 5 |
| (14) | Mica | 2 |
| (15) | Sphere silica | 3 |
| (16) | Glyceryl monostearate | 1 |
| (17) | Polyoxyethylene sorbitan monoisostearate | 0.5 |
| (18) | Cetyl alcohol | 0.4 |
| (19) | Batyl alcohol | 0.5 |
| (20) | Liquid paraffin | 5 |
| (21) | Dimethylpolysiloxane | 5 |
| (22) | 2-ethyl hexyl-paramethoxy cinnamate | 3 |
| (23) | Preservative | Adequate amount |
| (24) | Fragrance | Adequate amount |
| Ingredients 1 - 5 were stirred together thoroughly, to which ingredients 6 - 8 were added. Ingredients 9 - 15 were also combined and pulverized together, the mixture of which was added to the first mixture to be dispersed evenly. Finally, ingredients 16 - 25, which were dissolved by heat, were also added to the aforesaid mixture, which was consistently emulsified to form the oil-in-water type cosmetic. | | |

### Formula 5 Water-in-oil type emulsified cosmetic

| Ingredient | | Content (% by mass) |
|---|---|---|
| (1) | Silicon-treated nylon powder | 5 |
| (2) | Silicon-treated exfoliated composition produced using manufacturing method 1 according to the invention | 12 |
| (3) | Silicon-treated titanium oxide-coated barium sulfate | 4 |
| (4) | Silicon-treated titanium oxide | 10 |
| (5) | Silicon-treated iron oxide | 5 |
| (6) | Silicon-treated talc | 3 |
| (7) | Silicon-treated synthetic mica | 4 |
| (8) | Purified water | Residual |
| (9) | D ipropylene glycol | 8 |
| (10) | Decamethylcyclopentasiloxane | 25 |
| (11) | Dodecamethylcyclopentasiloxane | 15 |
| (12) | Dimethylpolysiloxane | 4 |
| (13) | Silicon resin | 2 |
| (14) | Polyether-modified silicon | 1.5 |
| (15) | Alkyl-polyether-modified silicon | 0.5 |
| (16) | Isostearic acid | 1 |
| (17) | Antioxidant | Adequate amount |
| (18) | Preservative | Adequate amount |
| Ingredients 10 - 18 were stirred evenly together and the pulverized mixture of ingredients 1 - 7 was added and dispersed in the first mixture. Ingredients 8 - 9 were also thoroughly combined together and added to the mixture, which was emulsified and set in a container to form the water-in-oil type emulsified cosmetic. | | |

### Formula 6 Eyeshadow

| Ingredient | | Content (% by mass) |
|---|---|---|
| (1) | Talc | Residual |
| (2) | Sericite | 6 |
| (3) | Synthetic mica | 12 |
| (4) | Powder of sphere PMMA particles | 3 |
| (5) | Barium sulfate | 2 |
| (6) | Exfoliated composition produced using manufacturing method 3 according to the invention | 8 |
| (7) | Iron oxide | 2 |
| (8) | Boron nitride | 3 |
| (9) | Squalane oil | 2 |
| (10) | Dimethylpolysiloxane | 2 |
| (11) | Sorbitan oleate | 1 |
| (12) | Preservative | Adequate amount |
| (13) | Fragrance | Adequate amount |
| Ingredients 1 - 8 were combined and pulverized together and the blend of ingredients 9 - 13 was stirred into the first mixture. The whole mixture was placed in a medium-size plate to form the eyeshadow. | | |

### Formula 7 Oil-based stick

| Ingredient | | Content (% by mass) |
|---|---|---|
| (1) | Carnauba wax | 1 |
| (2) | Candelilla wax | 2 |
| (3) | Ceresin | 10 |
| (4) | Squalane oil | Residual |
| (5) | Triiso glyceryl octanoate | 9 |
| (6) | Glyceryl diisostearate | 11 |
| (7) | High viscous dimethylpolysiloxane | 6 |
| (8) | Low viscous dimethylpolysiloxane | 5 |
| (9) | Silicon resin | 7 |
| (10) | Hydroxypropyl-β-cyclodextrin | 1 |
| (11) | Cholesteryl macadamiate | 1 |
| (12) | Sodium magnesium silicate | 0.5 |
| (13) | Hydrophobic silicon dioxide | 0.5 |
| (14) | Purified water | 2 |
| (15) | Barium sulfate | 2.5 |
| (16) | Exfoliated composition produced using manufacturing method 4 according to the invention | 8 |
| (17) | Iron oxide | 2 |
| (18) | Antioxidant | Adequate amount |
| (19) | Preservative | Adequate amount |
| (20) | Fragrance | Adequate amount |
| Ingredients 12 - 13 were dispersed in ingredient 11, which was heated to a temperature of 70°C. Ingredients 10 and 14 were thoroughly combined and stirred completely into the first mixture. Meanwhile, ingredients 1 - 9 were mixed together by heat, into which the first mixture was thoroughly stirred. Finally, ingredients 15 - 20 were added to the aforesaid mixture and stirred until they were completely dispersed in the mixture, after which the whole mixture was set in a container to form the oil-based stick. | | |

### Formula 8 Lipstick

| Ingredient | | Content (% by mass) |
|---|---|---|
| (1) | Polyethylene wax | 10 |
| (2) | Ceresin wax | 3 |
| (3) | lanoline | 17 |
| (4) | Polybutene | 18 |
| (5) | Octylmetoxycinnamate | 5 |
| (6) | Dimethylpolysiloxane | 12 |
| (7) | Ester oil | Residual |
| (8) | Titanium oxide | 5 |
| (9) | Red pigment 201 | 4.5 |
| (10) | Red pigment 202 | 1.1 |
| (11) | Red pigment 223 | 0.5 |
| (12) | Powder of sphere polyethylene particles | 3.5 |
| (13) | Exfoliated composition produced using manufacturing method 3 according to the invention | 8 |
| (14) | Antioxidant | Adequate amount |
| (15) | Fragrance | Adequate amount |
| Ingredients 1 - 7 were heated to dissolve at 80° and thoroughly combined together. Ingredients 8 - 11 were added to the solution and the mixture was kneaded by a three-roller machine until it formed slurry. The slurry was heated up to 80°C, to which ingredients 12 - 15 were added, and the slurry mixture was thoroughly stirred. The mixture was then placed in a lipstick mould to set. The lipstick was then completed. | | |

### Formula 9 Sunscreen agent

| Ingredient | | Content (% by mass) |
|---|---|---|
| (1) | Silicon-treated fine-particle zinc oxide | 11 |
| (2) | Silicon-treated exfoliated composition produced using manufacturing method 1 according to the invention | 12 |
| (3) | Decamethylcyclopentasiloxane | 18 |
| (4) | Fluorinated silicon resin | 6 |
| (5) | Powder of sphere silicon elastomer particles | 10 |
| (6) | Bentonite | 0.8 |
| (7) | Methylphenylpolysiloxane | 35 |
| (8) | Dimethylpolysiloxane | 7.2 |
| (9) | Preservative | Adequate amount |
| Ingredients 1 - 9 were mixed and dispersed by a dispersion mill, after which the mixture was set in a container together with stainless balls to form the above sunscreen agent. The sunscreen agent was shaken well before application. | | |

### Formula 10 Compact-type emulsified foundation

| Ingredient | | Content (% by mass) |
|---|---|---|
| (1) | Octylmetoxycinnamate | 5 |
| (2) | Sorbitan sesque-isostearate | 1.5 |
| (3) | Polyoxyethylene demethyl polysiloxane methyl | 1.5 |
| (4) | Distearyl dimethyl ammonium chloride | 0.2 |
| (5) | Dimethyl silicon (high-test) | 26.4 |
| (6) | Dimethyl silicon (low viscous) | 4.0 |
| (7) | Silicon-treated exfoliated composition produced using manufacturing method 2 according to the invention | 2.0 |
| (8) | Silicon-treated titanium oxide | 1.0 |
| (9) | Silicon-treated fine-particle titanium oxide | 6.0 |
| (10) | Silicon-treated sericite | 2.0 |
| (11) | Silicon-treated mica | 6.0 |
| (12) | Powder of sphere resin particles | 15.0 |
| (13) | Antioxidant | Adequate amount |
| (14) | Paraffin wax | 4.4 |
| (15) | Purified water | 15.0 |
| (16) | 1, 3-butylene glycol | 5.0 |
| (17) | Preservative | Adequate amount |
| (18) | Melilot extract | 5.0 |
| Ingredients 1 - 6 were heated up to 80°C and stirred thoroughly to disperse together with ingredients 7 - 11. Ingredient 12 was also added to disperse in the mixture. Ingredient 17 was added to ingredient 16, which was prepared separately, and ingredient 15 was also added to make a homogeneous solution. After ingredient 18 was added, the solution was heated to 70°C. The first mixture and the solution were then combined together to be emulsified in a homomixer. Ingredients 13 and 14 were added to the mixture and the entire mixture was deaerated, after which it was set and cooled in a container to form the compact-type emulsified foundation. | | |

### Formula 11 Nail enamel

| Ingredient | | Content (% by mass) |
|---|---|---|
| (1) | Cellulose nitrate | 12 |
| (2) | Alkyd resin | 10 |
| (3) | Acetyl tributyl citrate | 6 |
| (4) | Ethyl acetate | 30 |
| (5) | Butyl acetate | 20 |
| (6) | Ethyl alcohol | 5 |
| (7) | Red pigment 202 | 2 |
| (8) | Titanium oxide | 4 |
| (9) | Exfoliated composition produced using manufacturing method 3 according to the invention | 8 |
| (10) | Bentonite | 3 |
| Ingredients 1 - 3 and ingredients 7 - 8 were kneaded separately by a heat roller while ingredient 10 was added to the mixture of ingredients 4 - 6 to make a solution. The kneaded mixtures were added to the solution to be combined thoroughly. Once the mixture was of an even consistency, ingredient 9 was added to be dispersed and the whole mixture was set in a container to form the nail enamel. | | |

The highly iridescent titanium oxide composition, to which the present invention pertains, is a valuable optically-functional element that can be used with coatings, inks, plastics, catalysts etc. as well as with cosmetic products.

### Effect of the invention

As explained above, the present invention enabled the creation of the highly iridescent titanium oxide composition, which not only produces new and outstanding luster as well as color brightness within a recognized color range by interference colors but also manages to retain clear complementary colors.

The highly iridescent titanium oxide composition is a coating composition that forms a coating layer over the surface of a thin flake-shaped matrix, whose size is 50 - 800µm. The thickness of the coating layer is 0.05 - 0.6µm and it contains 70 - 95% of titanium composition by mass. This titanium oxide-contained coating layer is the highly iridescent titanium oxide composition, which is exfoliated from the coating composition. Furthermore, the blending of the exfoliated highly iridescent titanium oxide composition ("exfoliated composition" in the body of this specification) in cosmetic formulas provides cosmetic products with excellent effects; not only a smooth texture to the skin and natural transparency but also the control of pigmentation irregularities (blemish control effect) plus anti-UV and photochromic effects.

### Descriptions of the symbols

- 1.: Thin flake-shaped matrix
- 2.: Coating layers
- 2a.: Exfoliated composition

## Claims

1. A coating composition featuring a highly iridescent titanium oxide composition, which forms a coating layer over the surface of a thin flake-shaped matrix of a dimension 50 - 800µm and whose coating layer has a thickness of 0.05 - 0.6µm plus a content of titanium composition at a ratio 70 - 95% by weight.

2. The coating composition, which is a highly iridescent titanium oxide composition according to claim 1, wherein said thinly flake-shaped matrix consists of a material selected from natural mica, synthetic mica, glass flake, alumina flake and barium sulfate.

3. The coating composition, which is a highly iridescent titanium oxide composition according to claim 1, wherein said coating layer consists of a titanium composition; either titanium dioxide or titanium hydroxide.

4. The coating composition, which is a highly iridescent titanium oxide composition according to claim 1, wherein said titanium composition comprises particles of equal size.

5. The coating composition, which is a highly iridescent titanium oxide composition according to claim 1, wherein said coating layer contains a reinforcer whose content is 5 - 20% by weight.

6. The coating composition, which is a highly iridescent titanium oxide composition according to claim 5, wherein said reinforcer is either a metal oxide or a metal hydroxide, which contains one or two kinds or more of substances selected from iron, zinc, cobalt, nickel, lithium, sodium, bismuth, tungsten, tin, silica, alumina and zirconium.

7. The highly iridescent titanium oxide composition featuring an exfoliated composition produced by exfoliating a coating layer of titanium composition, which forms to a thickness of 0.05 - 0.6µm over the surface of a thinly flake-shaped matrix of a dimension 50 - 800µm.

8. The highly iridescent titanium oxide composition according to claim 7 wherein said exfoliated composition consists of either a titanium dioxide or a titanium hydroxide whose content is 70 - 99% by weight.

9. The highly iridescent titanium oxide composition according to claim 7 wherein said exfoliated composition consists of a reinforcer whose content is 1 - 20% by weight.

10. The highly iridescent titanium oxide composition according to claim 7 wherein said reinforcer is either a metal oxide or a metal hydroxide containing one or two kinds or more of substances selected from iron, zinc, cobalt, nickel, lithium, sodium, bismuth, tungsten, tin, silica, alumina and zirconium.

11. The coating composition, which is a highly iridescent titanium oxide composition according to claim7, wherein said exfoliated composition has a thickness of 0.05 - 0.6µm.

12. The highly iridescent titanium oxide composition according to claim 7 wherein said exfoliated composition has a luster degree of 55 - 90 at 60° which is measured using Gloss Checker IG-330 manufactured by Horiba International Corporation when one part of the exfoliated composition is mixed with thirty parts of clear acrylic lacquer and the mixture is applied onto the black side of a black-and-white hiding chart JISK5400 using a 4-mil applicator.

13. The highly iridescent titanium oxide composition according to claim 7 wherein said exfoliated composition produces colors within the layer of the applied surface by the interferential effect of light.

14. The highly iridescent titanium oxide composition according to claim 7 wherein said exfoliated composition forms within the layer of the applied surface with particles of equal size.

15. The highly iridescent titanium oxide composition according to claims 1 and 14 wherein said highly iridescent titanium oxide composition is a cosmetic ingredient.

16. The highly iridescent titanium oxide composition according to claims 1 and 14 wherein said highly iridescent titanium oxide composition is an ingredient for coatings and printing inks.

17. The highly iridescent titanium oxide composition according to claims 1 and 14 wherein said highly iridescent titanium oxide composition is an ingredient for external use.

18. The highly iridescent titanium oxide composition according to claims 1 and 14 wherein said highly iridescent titanium oxide composition is a viscous composition.

19. The highly iridescent titanium oxide composition according to claims 1 and 14 wherein said highly iridescent titanium oxide composition is a resin-mixed composition.

20. The highly iridescent titanium oxide composition according to claims 1 and 14 wherein said highly iridescent titanium oxide composition is a photocatalyst.

21. Four methods for the manufacture of highly iridescent titanium oxide composition, featuring exfoliation of a coating layer, which contains a titanium composition 70 - 95% by weight and which forms to a thickness of 0.05 - 0.6µm over the surface of a thinly flake-shaped matrix with a dimension of 50 - 800µm.

22. The methods for the manufacture of highly iridescent titanium oxide composition according to claim 21, wherein said coating composition contains a reinforcer whose content is 5 - 20% by weight.

23. The methods for the manufacture of highly iridescent titanium oxide composition according to claim 22, wherein said reinforcer is either a metal oxide or a metal hydroxide containing one or two kinds or more of substances selected from iron, zinc, cobalt, nickel, lithium, sodium, bismuth, tungsten, tin, silica, alumina and zirconium.

24. The first method for the manufacture of highly iridescent titanium oxide composition according to claim 21, wherein said exfoliated composition is exfoliated by hydrolysis using soluble sodium titanium solution or titanium alcoholate.

25. The second method for the manufacture of highly iridescent titanium oxide composition according to claim 21, wherein said exfoliated composition is exfoliated by a sequential process of filtering, water cleaning, drying and sintering at 300 - 800°C.

26. The third method for the manufacture of highly iridescent titanium oxide composition according to claim 21, wherein said exfoliated composition is exfoliated in alkaline solution.

27. The fourth method for the manufacture of highly iridescent titanium oxide composition according to claim 21, wherein said exfoliated composition is exfoliated in an alkaline solution at 8pH or more when it contains soluble carbonate, hydroxide and ammonium sodium.
